**Europäisches Patentamt**

**European Patent Office**

**Office européen des brevets**

(19)

(11)　**EP 1 434 141 A2**

(12)　**EUROPÄISCHE PATENTANMELDUNG**

(43) Veröffentlichungstag:
　　 **30.06.2004　Patentblatt 2004/27**

(51) Int Cl.[7]:　**G06F 17/00**

(21) Anmeldenummer: **03104552.9**

(22) Anmeldetag: **04.12.2003**

(84) Benannte Vertragsstaaten:
　　 **AT BE BG CH CY CZ DE DK EE ES FI FR GB GR
　　 HU IE IT LI LU MC NL PT RO SE SI SK TR**
　　 Benannte Erstreckungsstaaten:
　　 **AL LT LV MK**

(30) Priorität: **23.12.2002　DE 1026076**

(71) Anmelder: **Pulsion Medical Systems AG
　　 81829 München (DE)**

(72) Erfinder:
　　 • **Pfeiffer, Ulrich J.
　　　 81667, München (DE)**
　　 • **Burger, Thorsten
　　　 80804, München (DE)**

(74) Vertreter: **Kehl, Günther, Dipl.-Phys.
　　 Friedrich-Herschel-Strasse 9
　　 81679 München (DE)**

(54)　**Vorrichtung zur Bestimmung kardiovaskulärer Parameter**

(57)　　Die Vorrichtung (4) fragt über den Eingangskanal (5a) kontinuierlich den vom Drucksensor (6) in einer Arterie (7) gemessenen arteriellen Druck ab, welcher als näherungsweise dem Aortendruck entsprechende Meßgröße Pao aufzufassen ist. Grundsätzlich kann der arterielle Druck in der Aorta (2), deren Nähe oder im Arterienbaum gemessen werden. Als zweite Meßgröße fragt die Vorrichtung (4) über den Eingangskanal (5b) kontinuierlich den vom Drucksensor (8) in der Zentralvene (3) gemessenen zentralvenösen Druck CVP ab, welcher als näherungsweise dem intrathorakalen Druck ITP entsprechender Druck $P_{IT}$ betrachtet wird. Als dritte Meßgröße wird über den Eingangskanal (5c) eine die Thoraxausdehnung wiedergebende Meßgröße Z eingelesen. Mittels bekannter Algorithmen der Pulskonturanalyse berechnet die entsprechend programmtechnisch eingerichtete Vorrichtung (4) die Schlagvolumenvariation SW, wobei der nach der Formel

$$P_{transmural} = P_{ao} - f(C) \cdot PIT$$

berechnete transmurale Druck als maßgeblicher Druck verwendet wird. Der Volumenreaktionsindikator des Herzens CVRI wird nach der Formel

$$CVRI = k \cdot (SW / \Delta CVP)$$

für mechanische Positivdruckbeatmung bzw. nach der Formel

$$CVRI = I - m \cdot (\Delta CVP / SVV)$$

für Spontanatmung berechnet.

Fig. 3

**Beschreibung**

Die vorliegende Erfindung betrifft eine Vorrichtung zur Bestimmung kardiovaskulärer Parameter, insbesondere eine Vorrichtung zur kontinuierlichen Bestimmung eines die linksventrikuläre Pumpfunktion eines Patienten charakterisierenden Parameters sowie eine Vorrichtung zur kontinuierlichen Bestimmung des Volumenreaktionsindikators des Herzens.

Kardiovaskuläre Parameter müssen insbesondere bei kritisch kranken Patienten kontinuierlich überwacht werden, etwa während eines chirurgischen Eingriffs unter Narkose oder aufgrund eines kritischen Krankheitszustandes. Die linksventrikuläre Pumpfunktion charakterisierenden Parametern kommt hierbei besondere Bedeutung zu. Dabei ist es bekannt, diese mittels Pulskonturanalyse zu bestimmen, wofür bereits seit einiger Zeit technische Umsetzungen, beispielsweise das kommerziell erhältliche System PiCCO der Fa. Pulsion Medical Systems, verfügbar sind. Die zu bestimmenden Parameter werden dabei aus dem zeitlichen Verlauf eines arteriell gemessenen Druckes berechnet. Insbesondere die auf einem nicht-linearen Windkesselmodell beruhende Pulskonturanalyse, bei welcher, wie beispielsweise in DE 198 14 371 A1 beschrieben, für die sogenannte Compliance (Dehnbarkeit) der Aorta eine nichtlineare Funktion des aortennah gemessenen Druckes angesetzt wird, führt in der Regel zu sehr brauchbaren Meßergebnissen. Allerdings ist deren Aussagekraft bei mechanisch beatmeten Patienten mitunter noch verbesserungswürdig.

Ferner ist es bei der kardiovaskulären Überwachung von Intensivpatienten oft von entscheidender Bedeutung zu erkennen, ob einem kritischen Füllungszustand des Patienten durch Volumengaben oder medikamentös zu begegnen ist. Insbesondere bei künstlich beatmeten Patienten ist dies mitunter schwierig, da sich der Beatmungszustand auf die gemessenen Größen auswirkt. Dies ist im folgenden kurz erläutert.

Der Säugetierbrustkorb kann als eine Kammer variablen Volumens angesehen werden, welches sich aus den Teilvolumina des Herzens, der Lunge, Gefäßen außerhalb des Herzens und fixierten Geweben, wie Bindegewebe und Ösophagus, zusammensetzt. Das Thoraxvolumen ändert sich regelmäßig mit der Atmung oder mechanischen Beatmung; unter pathophysiologischen Bedingungen kann es sich aufgrund erhöhten abdominalen Drucks verändern, zudem durch äußere Druckeinwirkung, etwa während Tauchens etc. Betrachtet man ein variables Thoraxvolumen in zeitlicher Hinsicht, so enthält es Teilvolumina, welche sich sehr schnell ändern, beispielsweise innerhalb von Sekunden während eines Atmungs- oder Beatmungszyklus, wie das Gasvolumen innerhalb der Lungen und das Blutvolumen innerhalb großer Gefäße und innerhalb des Herzens, und Teilvolumina welche sich mit längeren Zeitkonstanten ändern, beispielsweise funktionelles Residualvolumen der Lungen aufgrund therapeutischer Einwirkung, wie bei Anwendung von konstantem positiven end-exspiratorischem Druck, eine Zunahme an extravasalem Lungenwasser (wenn sich ein Lungenödem bildet) und Zunahmen an pathologischen Teilvolumina (wie im Falle von Hämatothorax, Pneumothorax oder Pleuraerguß.

Seit langem ist bekannt, daß Wechselwirkungen des Herzens und der Lunge während der Atmung und insbesondere während der mechanischen Beatmung bestehen. Bei Spontanatmung tritt eingeatmete Luft aufgrund des negativen intrathorakalen Drucks (intrathoracic pressure ITP), welcher von den Brustwandmuskeln und dem Zwerchfell erzeugt wird, in die Lunge ein. Allerdings wird auch der venöse Zufluß des Blutes in den Brustbereich, oft als venöser Rückstrom bezeichnet, während des Einatmens erleichtert. Während des Ausatmens bei Spontanatmung wird der intrathorakale Druck wieder positiver, und hierdurch verläßt gleichzeitig Gas die Lunge, da der Druck innerhalb der Lunge Atmosphärendruck übersteigt, wohingegen der venöse Rückstrom zur gleichen Zeit verlangsamt wird. Das gleiche geschieht, wenn mechanische Beatmung durch Nachahmen der Spontanatmung mittels eines Beatmungsapparats vom Typ einer eisernen Lunge bewirkt wird.

Während der am häufigsten eingesetzten Form der mechanischen Beatmung, d.h. der Positivdruckbeatmung, wird die Einatmung durch Erzeugen eines positiven Drucks in den Luftwegen des Beatmungsapparats außerhalb der Lungen bewirkt. Beatmungsgas tritt in die Lunge ein, da der Gasdruck innerhalb der Lunge niedriger ist. Gas tritt in die Lunge ein, bis der Druck in den externen Luftwegen und der Gasdruck in den inneren Lungen-Luftwegen ein Gleichgewicht erreichen. Während dieses Einatmungsvorgangs werden die Lungen vergrößert, wodurch der intrathorakale Druck erhöht wird und hierdurch die großen Blutgefäße und das Herz selbst komprimiert werden. Physiologisch bedeutet dies, daß der venöse Rückstrom vermindert wird. Ausatmung findet statt aufgrund der Rückstellkräfte der Brustwand und der Lunge selbst und, in geringerem Grad, aufgrund des Gewichts der Brustwand selbst, wobei wieder eine Abnahme des intrathorakalen Drucks ITP und begleitend eine Zunahme des venösen Rückstroms stattfindet.

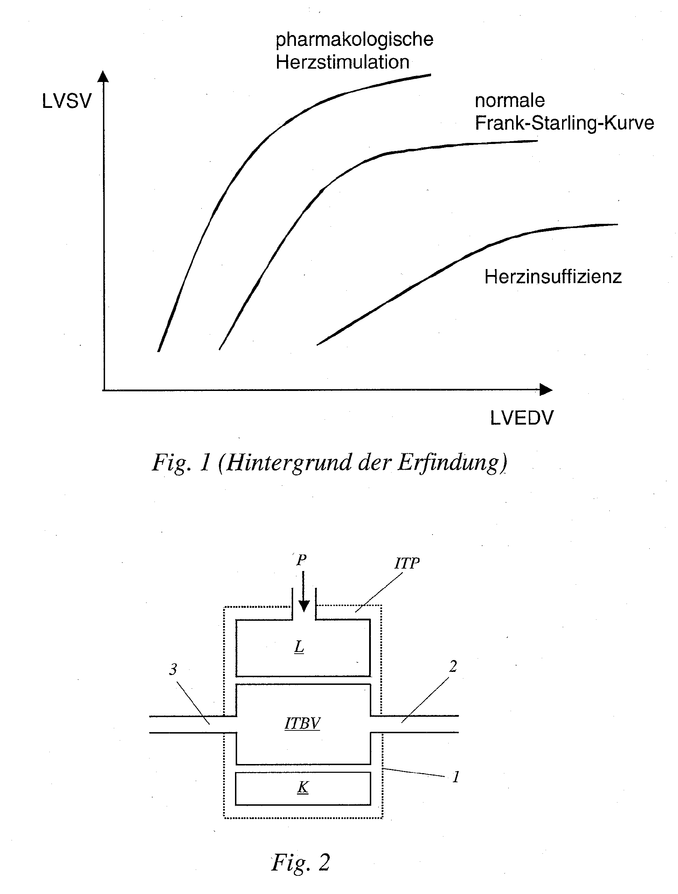

Die oben beschriebenen Änderungen des venösen Rückstroms, sowohl während Spontanatmung als auch während mechanischer Positivdruckbeatmung, haben direkte Auswirkung auf die Füllung des Herzens und - über den Frank-Starling-Mechanismus - auf den ventrikulären Ausstoß, d.h. das Schlagvolumen. Vereinfacht gesprochen beschreibt der Frank-Starling-Mechanismus eine Beziehung zwischen dem diastolischen Herzfüllvolumen und dem Schlagvolumen des Herzens. Je höher die Füllung des Herzens in der diastolischen Phase, desto größer ist das ausgestoßene Schlagvolumen. Diese Beziehung ist überwiegend linear für ein normales Herz und wird flacher, wenn ein sich normal kontrahierendes Herz in der diastolischen Phase überfüllt wird. Dies ist in Fig. 1 dargestellt, wo das linksventrikuläre Schlagvolumen LVSV schematisch qualitativ über dem linksventrikulären enddiastolischen Volumen LVEDV, welches im wesentlichen dem Füllstatus in der Diastole entspricht, aufgetragen ist. Die mittlere Kurve zeigt dabei den Normalverlauf. Verabreichen positiv-inotrop wirksamer Substanzen, beispielsweise von Adrenalin, d.h. Erhöhen der Kontraktionskraft des Herzens, verschiebt die Frank-Starling-Kurve nach links, wohingegen Bedingungen, welche mit akut oder chronisch verminderter Kontraktionskraft des Herzens einhergehen, die Steigung vermindern und eine Verschiebung der Kurve nach rechts bewirken. Entsprechend unterschiedlich kann bei gleichem gemessenen enddiastolischen Volumen die Reaktion auf Volumengaben ausfallen.

Bei Spontanatmung oder mechanischer Positivdruckbeatmung verursachen die Auswirkungen auf die Füllung des Herzens, hauptsächlich die Füllung des rechten Ventrikels, einen variierenden Schlagvolumenausstoß des rechten Ventrikels, der sich nach Durchströmen der Lunge wiederum auf die Füllung und den Ausstoß des linken Ventrikels auswirkt. Schließlich können diese Veränderungen der Füllung des Herzens anhand von periodischen Schwankungen der Aortendruckkurve oder arteriellen Druckkurve erfaßt werden, welche direkt die Veränderungen im linksventrikulären Volumenausstoß widerspiegeln.

Eine Vorrichtung zur Beurteilung der Notwendigkeit augenblicklicher Volumengaben, bzw. wie ein beatmeter Patient auf die intravenöse Verabreichung von Volumensubstitutionsmitteln anspricht, ist in EP 0 666 056 B1 offenbart, wobei die systolische Druckvariation als Indikator für die Volumenempfänglichkeit des Patienten dient. Hierbei ist es allerdings erforderlich, das Atemzugsvolumen bzw. den Beatmungsdruck am Beatmungsgerät zu erfassen und mit der hämodynamischen Antwort zu vergleichen, um Aussagen treffen zu können. Eine umfängliche Berücksichtigung aller Wechselwirkungen zwischen Beatmungszustand und gemessenen kardiovaskulären Größen ist nicht möglich. Zudem erlaubt die bekannte Vorrichtung keine Aussagen bei Rückführung des Patienten in die Spontanatmung.

Vor dem hintergrund der oben geschilderten Problematik ist es Aufgabe der vorliegenden Erfindung, eine Vorrichtung zu schaffen, welche die Bestimmung eines die linksventrikuläre Pumpfunktion eines Patienten charakterisierenden Parameters ermöglicht, ohne daß die Aussagekraft des Meßergebnisses durch Auswirkungen der Beatmung und wechselnde Beatmungszustände getrübt wird. Ferner ist es Aufgabe der vorliegenden Erfindung, eine Vorrichtung zu schaffen, welche die Bestimmung eines Parameters vorsieht, der die Volumenreaktion des Herzens, d.h. die Empfänglichkeit für Volumengaben, auch bei unterschiedlichen Beatmungszuständen zuverlässig zu beurteilen gestattet.

Gemäß einem Aspekt dieser Erfindung wird diese Aufgabe durch eine Vorrichtung zur kontinuierlichen Bestimmung eines die linksventrikuläre Pumpfunktion eines Patienten charakterisierenden Parameters gemäß Anspruch 1 gelöst. Bevorzugte Ausführungsformen umfassen Vorrichtungen gemäß den Ansprüchen 2 bis 10.

Gemäß einem weiteren Aspekt dieser Erfindung wird die Aufgabe durch eine Vorrichtung zur kontinuierlichen Bestimmung des Volumenreaktionsindikators des Herzens gemäß Anspruch 11 gelöst.

Die durch die Herz/Lungen-Wechselwirkung verursachten periodischen Schwankungen des rechts- oder linksventrikulären Schlagvolumens, oder erfaßte periodische Schwankungen eines anderen physiologisches Signals, welche den periodischen Schwankungen des rechts- oder linksventrikulären Schlagvolumen-Ausstoßes entsprechen oder diese widerspiegeln, wie in der Arteria pulmonalis, der Aorta oder dem Arterienbaum gemessene Druckkurven, oder periodische Schwankungen des Signals der plethysmographischen Pulsoximetrie, werden verwendet, um die Reaktion des Herzens auf sich ändernde Füllungsbedingungen des Herzens, d.h. die Volumenvorlast des Herzen, zu diagnostizieren. Wie bereits oben beschrieben, bedeuten diese von der Atmung bzw. Beatmung abhängigen periodischen Schwankungen, daß das Herz auf Änderungen der Vorlast des Herzens reagiert, wobei diese durch Auswirkungen des sich ändernden intrathorakalen Druckes auf das Volumen des intrathorakalen Niederdruckkapazitätssystems (intrathoracic low pressure capacitance system ITLPCS), bestehend aus Venae cava superior und inferior, rechtem Atrium, rechtem Ventrikel in der Diastole, pulmonalem Gefäßsystem und linkem Atrium, hervorgerufen werden. Das ITLPCS ist durch einen niedrigen mittleren intravasalen Druck bei relativ großem intravasalen Volumen gekennzeichnet. Im ITLPCS ist der Zusammenhang zwischen Volumen und Druck nichtlinear, das bedeutet, daß bei geringem Volumen der Druck sehr niedrig ist, wohingegen der mittlere intravasale Druck mit zunehmendem Volumen mit einer Progression zunimmt. In anderen Worten haben bei Hypovolämie Veränderungen des Volumens des ITLPCS wenig Auswirkungen auf den Druck, während bei Hypervolämie jegliche Veränderungen des Volumens des ITLPCS große Auswirkungen auf den mittleren Druck des ITLPCS haben. Jegliche Veränderung des intrathorakalen Druckes, wobei es sich um den das ITLPCS umgebenden Druck handelt, wird aufgrund der sehr hohen Compliance (Nachgiebigkeit) der im ITLPCS enthaltenen Gefäßstrukturen direkt auf den intravasalen Druck innerhalb des ITLPCS übertragen. Der transmurale Druck des ITPLCS kann als die Differenz zwischen dem intravasalen Druck und dem intrathorakalen Druck abgeschätzt werden. Der effektive transmurale Druck im ITLPCS bestimmt die aktuelle Gefäßdehnung und somit das Volumen innerhalb des ITPLCS.

Folglich verursacht ein gleichbleibender, beispielsweise mechanischer, Atemzug gleichbleibender Tiefe die gleiche Änderung des intrathorakalen Drukkes, besitzt jedoch in Abhängigkeit vom Volumenzustand des ITPLCS unterschiedliche Auswirkungen auf die Vorlast des Herzens und damit das Schlagvolumen. Der Hauptanteil des ITLPCS besteht aus den Venae cava superior und inferior, dem rechten Atrium und dem rechten Ventrikel in der Diastole und weist zugleich den niedrigsten mittleren intravasalen Druck auf. Daher beeinflussen durch Atmung bzw. Beatmung verursachte Auswirkungen auf den intrathorakalen Druck stark das rechtsventrikuläre enddiastolische Volumen (RVEDV) und im Anschluß das rechtsventrikuläre Schlagvolumen (RVSV), jedoch in viel geringerem Maße direkt das linksventrikuläre enddiastolische Volumen (LVEDV) und das linksventrikuläre Schlagvolumen (LVSV). Zeitlich versetzt können jedoch diese Auswirkungen auf das rechtsventrikuläre Schlagvolumen nach Durchlaufen der Lunge auch in Veränderungen des linksventrikulären enddiastolischen Volumens und des linksventrikulären Schlagvolumens bemerkt werden.

Daher wird Atmung in der gleichen Größenordnung bei Anwesenheit von Hypovolämie eine viel größere Auswirkung auf den Verlauf des linksventrikulären Schlagvolumens zeigen als bei Hypervolämie, vorausgesetzt der linke Ventrikel reagiert auf Änderungen der Vorlast. Die Gesamtheit dieser Beziehungen kann mit Hilfe der erfindungsgemäßen Vorrichtung klinisch verwendet werden, um die Volumenreaktion des linken Ventrikels zu untersuchen. Als klinische Konsequenz wird man einem Patienten Volumen zuführen, dessen linkes Herz volumenempfänglich ist, das bedeutet, dessen Herz in einem steileren Abschnitt seiner momentanen Frank-Starling-Kurve arbeitet, und so sein Herzzeitvolumen (cardiac output CO) optimieren. Man wird dagegen Volumengaben bei einem Patienten vermeiden, dessen linkes Herz nicht volumenempfänglich ist, da sein Herz im flachen Abschnitt seiner momentanen Frank-Starling-Kurve arbeitet, und stattdessen versuchen, sein Herzzeitvolumen durch Verabreichen positiv inotropisch wirkender Substanzen zu optimieren, um seine Funktionskurve in eine steilere Form zu bewegen (Linksverschiebung, vgl. Fig. 1).

Diese Information ist sehr wertvoll für den Anästhesisten oder Intensivmediziner und kann einfach und vollautomatisch von einem nach Art der erfindungsgemäßen Vorrichtung gestalteten Überwachungssystem erhalten werden, welches kontinuierlich das Schlagvolumen oder eine beliebige Variable liefert, welche das Schlagvolumen widerspiegelt, sowie die Information, welche Art und Phase der Atmung oder mechanischen Beatmungsform vorherrscht bzw. angewandt wird, und wie der intrathorakale Druck von der entsprechenden Atmung oder Beatmungsmaßnahme beeinflußt wird.

Dieser Ansatz bietet den Vorteil, daß die für die Untersuchung der Volumenreaktion des Herzens (cardiac volume responsivenes) verwendete Herz-Lungen-Wechselwirkung sowohl bei Spontanatmung als auch bei mechanischer Beatmung auf die dadurch bedingte Veränderung des intrathorakalen Druckes normiert werden kann, wobei das Atemzugvolumen nicht notwendigerweise standardisiert sein oder einem gewissen Einprägungsmuster folgen muß sondern von jeglicher Größenordnung sein kann.

Es ist dagegen wenig sinnvoll zu versuchen, auf die Atmung selbst beschreibende Größen zu normieren, da der Grad deren Auswirkung auf das ITLPCS von der Compliance (Dehnbarkeit) des Atmungstraktes selbst, von der Compliance der Brustwand, und von der Anwesenheit eines raumeinnehmenden Vorgangs mit niedriger Zeitkonstante, wie beispielsweise einem Lungenödem, Pneumo- und Hämatothorax, Pleuraerguß, abhängt. Das Tidalvolumen (= Atemzugsvolumen) und alle obengenannten Faktoren tragen zu der Veränderung des intrathorakalen Druckes bei, welcher das ITPLCS direkt beeinflußt. Daher werden durch Atmung/ Beatmung bedingte Effekte am besten durch Berücksichtigung der einhergehenden Veränderungen des intrathorakalen Druckes normiert. Phasenbezogene Änderungen des intrathorakalen Druckes können direkt aus der Änderung des kontinuierlich aufgezeichneten Zentralvenösen Druckes (central venous pressure CVP) abgeleitet werden, wenn Änderungen der thorakalen Abmessungen gleichzeitig aufgezeichnet werden, oder, nur anwendbar bei mechanischer Positivdruckbeatmung, wenn die zeitliche Charakteristik und die Form der mechanischen Beatmung von dem - somit zeitliche Verschiebung vermeidend - nahe am Patienten gemessenen Atemwegsdruck abgeleitet werden.

Bevorzugte Ausführungsformen umfassen Vorrichtungen gemäß den Ansprüchen 12 bis 29.

Ein Ausführungsbeispiel der Erfindung sowie deren Funktionsprinzip wird anhand der beigefügten schematischen Zeichnungen nachfolgend näher erläutert.

Fig. 1    zeigt zum Hintergrund der Erfindung eine Frank-Starling-Kurve sowie deren Veränderung bei pharmakologischer Herzstimulation bzw. Herzinsuffizienz.

Fig. 2    verdeutlicht die grundsätzliche Wechselwirkung unterschiedlicher Volumina mit dem intrathorakalen Druck.

Fig. 3    zeigt eine erfindungsgemäße Vorrichtung in der Anwendung am Patienten.

Fig. 4a    zeigt schematisch die zeitlichen Verläufe der für die Berechnung des Volumenreaktionsindikators maßgeblichen Größen bei Positivdruckbeatmung.

Fig. 4b    zeigt schematisch die zeitlichen Verläufe der für die Berechnung des Volumenreaktionsindikators maßgeblichen Größen bei Spontanatmung.

Fig. 5a    zeigt schematisch den Verlauf des Volumenreaktionsindikators in Abhängigkeit des enddiastolischen Volumens bei Positivdruckbeatmung.

Fig. 5b    zeigt schematisch den Verlauf des Volumenreaktionsindikators in Abhängigkeit des enddiastolischen Volumens bei Spontanatmung.

In Fig. 2 sind die vom intrathorakalen Druck ITP beeinflußten bzw. diesen beeinflussenden Volumina sowie weitere, im Zusammenhang mit der Erfindung bedeutsame, charakteristische Drücke schematisch dargestellt. Der intrathorakale Druck ITP wirkt innerhalb der Thoraxwand 1 auf das intrathorakale Blutvolumen ITBV welches sich aus dem oben erwähnten intrathorakalen Niederdruckkapazitätssystem ITLPCS und dem linksventrikulären enddiastolischen Volumen LVEDV zusammensetzt. Weitere Volumina innerhalb der Thoraxwand 1 sind das gasgefüllte Lungenvolumen L sowie gegebenenfalls zusätzliche, während einer Meßperiode als konstant anzusehende Volumina K, beispielsweise extravasales Lungenwasser, Pleuraerguß, Hämatothorax etc. In der Aorta 2 herrscht der Aortendruck AP und in der Zentralvene 3 der zentralvenöse Druck CVP (central venous pressure). Bei künstlicher Beatmung liegt zudem der äußere Druck P an den Luftwegen an.

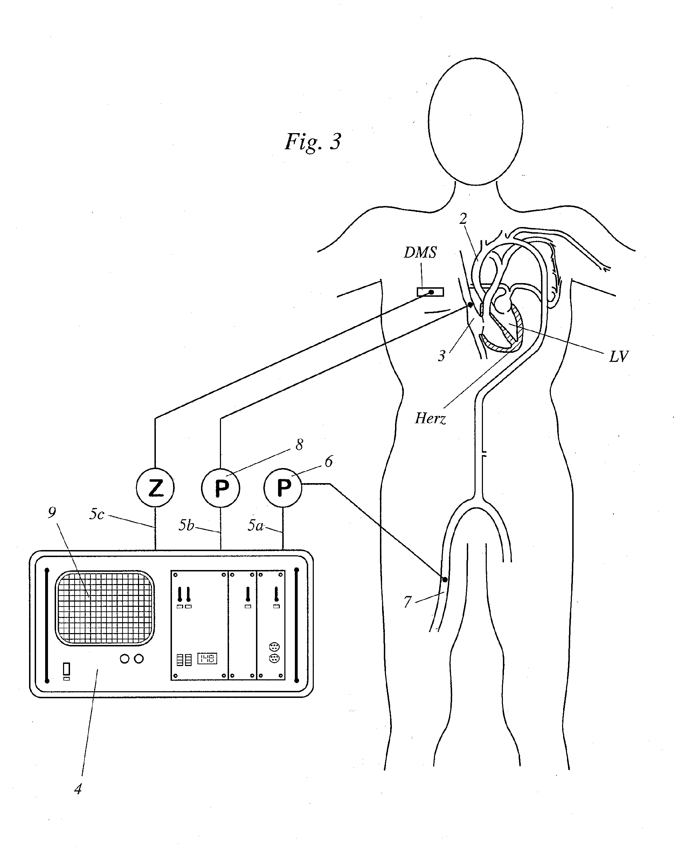

Die in Fig. 3 dargestellte erfindungsgemäße Vorrichtung 4 fragt über den Eingangskanal 5a kontinuierlich den vom Drucksensor 6 in einer Arterie 7 gemessenen arteriellen Druck ab, welcher als zumindest näherungsweise dem Aortendruck entsprechende Meßgröße Pao aufzufassen ist. Grundsätzlich kann der arterielle Druck in der Aorta 2, deren Nähe oder im Arterienbaum gemessen werden. Als zweite Meßgröße fragt die Vorrichtung 4 über den Eingangskanal 5b kontinuierlich den vom Drucksensor 8 in der Zentralvene 2 gemessenen zentralvenösen Druck CVP ab, welcher als näherungsweise dem intrathorakalen Druck ITP entsprechender Druck PIT betrachtet wird. Als dritte Meßgröße wird über den Eingangskanal 5c eine die Thoraxausdehnung wiedergebende Meßgröße Z eingelesen. Die Messung kann beispielsweise über Dehnungsmeßstreifen DMS erfolgen, aber auch die Messung einer anderen Größe, welche unmittelbar oder mittelbar die Thoraxausdehnung in absoluten oder relativen Werten wiedergibt, ist möglich.

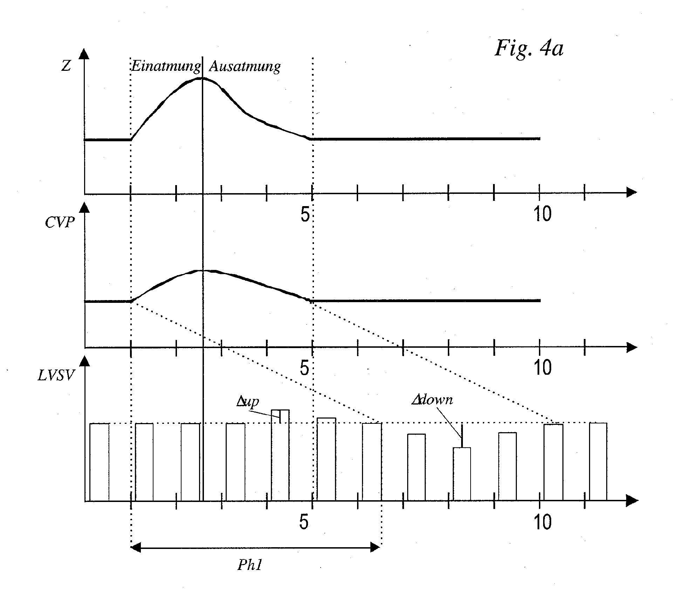

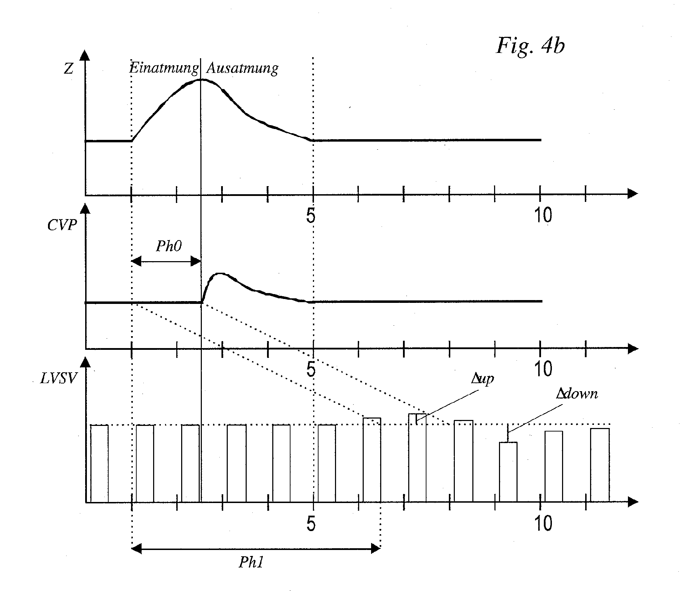

Typische erhaltene zeitliche Verläufe für die Thoraxdimension Z (jeweils oberes Diagramm) und den zentralvenösen Druck CVP sind für mechanische Positivdruckbeatmung in Fig. 4a und für Spontanatmung in Fig. 4b dargestellt. Die Abszisse ist dabei die Zeitachse mit Pulszyklen des Herzens als Achseinheit.

Mittels bekannter Algorithmen der Pulskonturanalyse berechnet die entsprechend programmtechnisch eingerichtete Vorrichtung 4 das Schlagvolumen LVSV (jeweils unteres Diagramm in Figuren 4a und 4b) des linken Ventrikels LV sowie die Schlagvolumenvariation SVV und ggf. weitere gewünschte kardiovaskuläre Parameter. Dabei wird der nach der Formel

$$P_{transmural} = P_{ao} - f(C) \cdot P_{IT}$$

berechnete transmurale Druck als maßgeblicher Druck verwendet. Die Korrekturfunktion f(C) ist dabei eine Fuktion der Compliance C des arteriellen Systems bzw. primär der Aorta 2, wobei die Compliance vorzugsweise nach einem nichtlinearen Windkesselmodell bestimmt ist, und die Korrekturfunktion f(C) beispielsweise die Form

$$f(C) = 1 - \exp(-a \cdot C)$$

mit a als Anpassungsparameter aufweisen kann, jedenfalls aber mit zunehmender Compliance monoton wächst und Werte zwischen 0 und 1 annehmen kann.

Zu berücksichtigen ist eine Phasenverschiebung Ph1 aufgrund des transpulmonalen und leftventrikulären Weges. Statt der aus dem Schlagvolumen LVSV berechneten Schlagvolumenvariation SVV können alternativ auch jeweils nur die Abweichungen des Schlagvolumens nach oben $\Delta up$ bzw. unten $\Delta down$ betrachtet werden. $\Delta up$ und $\Delta down$ können auch für die Berechnung weiterer gewünschter kardiovaskulärer Parameter benötigt werden.

Ob mechanische Beatmung oder Spontanatmung vorliegt, ist an der Phasenverschiebung Ph0 zwischen dem Beginn des Anstiegs des Verlaufs der Thoraxdimension Z, d.h. beginnender Thoraxexpansion, und dem Beginn des Anstiegs des Verlaufs des zentralvenösen Drucks CVP erkennbar. Eine solche Phasenverschiebung Ph0 ist nämlich nur bei Spontanatmung (Fig. 4b) merklich vorhanden, nicht jedoch bei mechanischer Positivdruckatmung (Fig. 4a). Als Kriterium dienen kann stattdessen jedoch auch die weniger ausgeprägte Phasenverschiebung der lokalen Maxima der Verläufe der Thoraxdimension Z und des zentralvenösen Drucks CVP.

Anhand des erläuterten Kriteriums wird ausgewählt, ob der Volumenreaktionsindikator des Herzens CVRI (cardiac volume responsiveness indicator)nach der Formel

$$CVRI = k \cdot (SVV / \Delta CVP)$$

für mechanische Positivdruckbeatmung oder nach der Formel

$$CVRI = I - m \cdot (\Delta CVP / SVV)$$

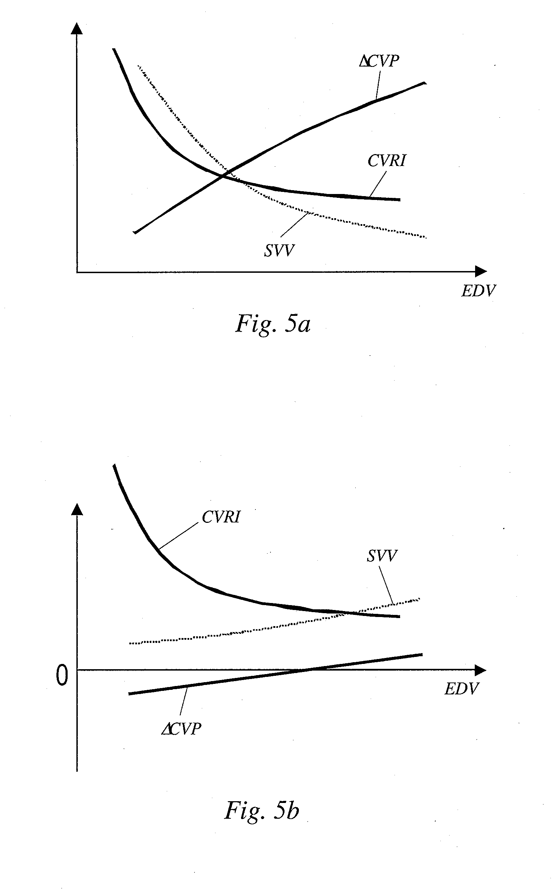

für Spontanatmung berechnet wird, wobei für $\Delta CVP$ die Variation des zentralvenösen Druckes während eines Atmungszyklus einzusetzen ist. Die jeweils in Abhängigkeit des enddiastolischen Volumens EDV erhaltenen Verläufe sind in Fig. 5a (Positivdruckbeatmung) und Fig. 5b (Spontanatmung) dargestellt.

Dabei sind k, I und m Anpassungsparameter. In der Regel wird es sich hier um geschätzte oder experimentell ermittelte Konstanten handeln, jedoch können auch geeignete Anpassungsfunktionen eingesetzt werden, etwa mit dem Ziel, daß beide Funktionen in ihrem Anwendungsbereich bei gleichem enddiastolischen Volumen EDV für den Volumenreaktionsindikator des Herzens CVRI des Patienten den jeweils gleichen Wert liefern.

Der berechnete Volumenreaktionsindikator CVRI wird auf dem Monitor 9 zusammen mit dem ebenfalls berechneten Herzeitvolumen CO ausgegeben und kann bei nicht zufriedenstellendem Füllzustand des Patienten dem behandelnden Arzt als Orientierung dienen, ob Volumengaben erforderlich sind, oder eine medikamentöse Maßnahme vorzuziehen ist.

**Patentansprüche**

1. Vorrichtung zur kontinuierlichen Bestimmung eines die linksventrikuläre Pumpfunktion eines Patienten charakterisierenden Parameters, aufweisend

   - einen ersten Eingangskanal (5a) zum kontinuierlichen Einlesen einer unmittelbar von der linksventrikulären Pumpfunktion abhängigen, veränderlichen, physiologischen ersten Meßgröße und
   - eine Auswerteeinheit zur Berechnung des die linksventrikuläre Pumpfunktion charakterisierenden Parameters

   **dadurch gekennzeichnet,**
   **daß** die Vorrichtung (4) ferner einen zweiten Eingangskanal (5b) zum kontinuierlichen Einlesen einer den intrathorakalen Druck ITP des Patienten zumindest näherungsweise wiedergebenden oder von diesem direkt abhängigen, veränderlichen, physiologischen zweiten Meßgröße aufweist, wobei die Auswerteeinheit programmtechnisch für die Berechnung des die linksventrikuläre Pumpfunktion des Patienten charakterisierenden Parameters aus der ersten Meßgröße unter Verwendung einer mit der zweiten Meßgröße gebildeten Korrekturfunktion eingerichtet ist.

2. Vorrichtung gemäß Anspruch 1, **dadurch gekennzeichnet, daß** der erste Eingangskanal (5a) zum Abfragen eines Druckmeßumformersignals ausgelegt ist, die erste Meßgröße zumindest näherungsweise dem Aortendruck des Patienten entspricht, und

die programmtechnische Einrichtung der Auswerteeinheit die Berechnung des die linksventrikuläre Pumpfunktion des Patienten charakterisierenden Parameters mittels Pulskonturanalyse vorsieht.

3. Vorrichtung gemäß Anspruch 2, **dadurch gekennzeichnet, daß** die zumindest näherungsweise dem Aortendruck entsprechende erste Meßgröße ein arterieller Druck ist.

4. Vorrichtung gemäß Anspruch 3, **dadurch gekennzeichnet, daß** der arterielle Druck ein aortennah gemessener Druck ist.

5. Vorrichtung gemäß einem der Ansprüche 2-4, **dadurch gekennzeichnet, daß** der Pulskonturanalyse ein nichtlineares Windkesselmodell zugrunde gelegt ist.

6. Vorrichtung gemäß einem der Ansprüche 2-5, **dadurch gekennzeichnet, daß** die Korrekturfunktion die Form

$$P_{transmural} = P_{ao} - f(C) \cdot P_{IT}$$

besitzt, wobei
für $P_{ao}$ die zumindest näherungsweise dem Aortendruck entsprechende erste Meßgröße einzusetzen ist,
für $P_{IT}$ die den intrathorakalen Druck ITP zumindest näherungsweise wiedergebende zweite Meßgröße einzusetzen ist, und
f(C) eine von der Compliance C des arteriellen Gefäßsystems oder der Aorta abhängige Funktion ist, welche mit zunehmender Compliance monoton wächst und Werte zwischen 0 und 1 annehmen kann,
und wobei der transmurale Druck Ptransmural als maßgeblicher Druck in die Pulskonturanalyse eingeht.

7. Vorrichtung gemäß Anspruch 6, **dadurch gekennzeichnet, daß** die Funktion f(C) die Form

$$f(C) = 1 - exp(-a \cdot C)$$

besitzt, worin
$exp(-a \cdot C)$ die Exponentialfunktion mit dem Argument $-a \cdot C$ bedeutet, C die Compliance des arteriellen Gefäßsystems oder der Aorta (2) ist, und a eine geschätzte oder experimentell ermittelte Konstante ist.

**8.** Vorrichtung gemäß Anspruch 7, **dadurch gekennzeichnet, daß** eine Abhängigkeit der Compliance C vom transmuralen Druck Ptransmural angenommen wird, und die iterative Berechnung beider Größen vorgesehen ist.

**9.** Vorrichtung gemäß einem der vorangehenden Ansprüche, **dadurch gekennzeichnet, daß** der zweite Eingangskanal (5b) zum Abfragen eines Druckmeßumformersignals ausgelegt ist, und die zweite Meßgröße zumindest näherungsweise dem zentralvenösen Druck CVP des Patienten entspricht.

**10.** Vorrichtung gemäß einem der vorangehenden Ansprüche, **dadurch gekennzeichnet, daß** der die linksventrikuläre Pumpfunktion eines Patienten charakterisierende Parameter das Herzeitvolumen oder das Schlagvolumen oder direkt aus diesen berechenbar ist.

**11.** Vorrichtung zur kontinuierlichen Bestimmung des Volumenreaktionsindikators des Herzens, aufweisend

- einen ersten Eingangskanal (5a) zum kontinuierlichen Einlesen einer unmittelbar von der linksventrikulären Pumpfunktion des Patienten abhängigen, veränderlichen, physiologischen ersten Meßgröße,
- einen zweiten Eingangskanal (5b) zum kontinuierlichen Einlesen einer den intrathorakalen Druck ITP des Patienten zumindest näherungsweise wiedergebenden, veränderlichen, physiologischen zweiten Meßgröße,
- einen dritten Eingangskanal (5c) zum kontinuierlichen Einlesen einer unmittelbar vom Beatmungszustand des Patienten abhängigen dritten Meßgröße, sowie
- eine Auswerteeinheit zur Berechnung des Volumenreaktionsindikators des Herzens,

wobei die Auswerteeinheit programmtechnisch so eingerichtet ist, daß sie anhand der zweiten und dritten Meßgröße eine für den momentanen Beatmungszustand des Patienten anwendbare Funktion auswählt und mittels dieser Funktion den Volumenreaktionsindikator des Herzens aus der ersten und der zweiten Meßgröße berechnet.

**12.** Vorrichtung gemäß Anspruch 11, **dadurch gekennzeichnet, daß** die erste Meßgröße die durch Röntgendensitometrie, Positronenemissionstomographie, transösophageale oder transthorakale Echokardiographie oder ein sonstiges zwei- oder dreidimensionales bildgebendes Verfahren ermittelte Größe des linken Ventrikels ist.

**13.** Vorrichtung gemäß Anspruch 11, **dadurch gekennzeichnet, daß** die von der linksventrikulären Pumpfunktion des Patienten abhängige erste Meßgröße eine durch Plethysmographie, elektrische Impedanz- oder Leitfähigkeitsmessung, Ultraschallmessung, Druckmessung in der Arteria pulmonalis oder direkte Flußmessung ermittelte Größe ist.

**14.** Vorrichtung gemäß Anspruch 11 **dadurch gekennzeichnet, daß** der erste Eingangskanal (5a) zum Abfragen eines Druckmeßumformersignals ausgelegt ist, und die erste Meßgröße zumindest näherungsweise dem Aortendruck des Patienten entspricht.

**15.** Vorrichtung gemäß einem der Ansprüche 11-14, **dadurch gekennzeichnet, daß** die programmtechnische Einrichtung der Auswerteeinheit die Berechnung der Schlagvolumenvariation SVV, systolischen Druckvariation SPV, Pulsdruckvariation PPV oder vergleichbaren Größe aus der zeitlichen Veränderung der ersten Meßgröße vorsieht.

**16.** Vorrichtung gemäß Anspruch 14, **dadurch gekennzeichnet, daß** die Auswerteeinheit programmtechnisch für die Berechnung der Schlagvolumenvariation SVV, systolischen Druckvariation SPV, Pulsdruckvariation PPV oder vergleichbaren Größe aus der ersten Meßgröße mittels Pulskonturanalyse unter Verwendung einer mit der zweiten Meßgröße gebildeten Korrekturfunktion eingerichtet ist.

**17.** Vorrichtung gemäß Anspruch 16, **dadurch gekennzeichnet, daß** die Korrekturfunktion die Form

$$P_{transmural} = P_{ao} - f(C) \cdot P_{IT}$$

besitzt, wobei
für $P_{ao}$ die zumindest näherungsweise dem Aortendruck entsprechende erste Meßgröße einzusetzen ist,
für $P_{IT}$ die den intrathorakalen Druck ITP zumindest näherungsweise wiedergebende zweite Meßgröße einzusetzen ist, und
f(C) eine von der Compliance C des arteriellen Gefäßsystems oder der Aorta (2) abhängige Funktion ist, welche mit zunehmender Compliance monoton wächst und Werte zwischen 0 und 1 annehmen kann,
und wobei der transmurale Druck Ptransmural als maßgeblicher Druck in die Pulskonturanalyse eingeht.

**18.** Vorrichtung gemäß Anspruch 17, **dadurch gekennzeichnet, daß** die Funktion f(C) die Form

$$f(C) = 1 - \exp(-a \cdot C)$$

besitzt, worin
exp $(-a \cdot C)$ die Exponentialfunktion mit dem Argument $-a \cdot C$ bedeutet, C die Compliance des arteriellen Gefäßsystems oder der Aorta (2) ist, und a eine geschätzte oder experimentell ermittelte Konstante ist.

19. Vorrichtung gemäß Anspruch 18, **dadurch gekennzeichnet, daß** eine Abhängigkeit der Compliance C vom transmuralen Druck Ptransmural angenommen wird, und die iterative Berechnung beider Größen vorgesehen ist.

20. Vorrichtung gemäß einem der Ansprüche 11 -19, **dadurch gekennzeichnet, daß** die Auswahl der für den momentanen Beatmungszustand des Patienten anwendbaren Funktion die Auswahl zwischen einer für künstliche Beatmung des Patienten gültigen ersten Funktion und einer für Spontanatmung des Patienten gültigen zweiten Funktion umfaßt.

21. Vorrichtung gemäß Anspruch 20, **dadurch gekennzeichnet, daß** die für künstliche Beatmung des Patienten gültige erste Funktion die Form

$$CVRI = k \cdot (XXV / \Delta P_{IT})$$

besitzt, und die für Spontanatmung des Patienten gültige zweite Funktion die Form

$$CVRI = I - m \cdot (\Delta P_{IT} / XXV)$$

besitzt, wobei
CVRI der Volumenreaktionsindikator des Herzens ist,
für XXV die Schlagvolumenvariation SVV, systolischen Druckvariation SPV, Pulsdruckvariation PPV oder vergleichbare die Variation der linksventrikulären Pumpfunktion beschreibende Größe einzusetzen ist,
für ΔPIT die Variation der den intrathorakalen Druck ITP zumindest näherungsweise wiedergebenden zweiten Meßgröße während eines Atmungszyklus einzusetzen ist,
und k, l und m Anpassungsparameter sind.

22. Vorrichtung gemäß Anspruch 21, **dadurch gekennzeichnet, daß** die Anpassungsparameter k, l und m Anpassungsfunktionen sind.

23. Vorrichtung gemäß Anspruch 21, **dadurch gekennzeichnet, daß** die Anpassungsparameter k, l und m geschätzte oder experimentell ermittelte Konstanten sind.

24. Vorrichtung gemäß einem der Ansprüche 21-23, **dadurch gekennzeichnet, daß** die Anpassungsparameter k, I und m so gewählt sind, daß beide Funktionen in ihrem Anwendungsbereich bei gleichem enddiastolischen Volumen für den Volumenreaktionsindikator des Herzens CVRI des Patienten den jeweils gleichen Wert liefern.

25. Vorrichtung gemäß einem der Ansprüche 20-24, **dadurch gekennzeichnet, daß** die programmtechnische Einrichtung der Auswerteeinheit die Ermittlung des Beginns der Einatmung des Patienten anhand des zeitlichen Verlaufs der dritten Meßgröße vorsieht,
und die Bestimmung, ob die für künstliche Beatmung des Patienten gültige erste Funktion oder die für Spontanatmung des Patienten gültige zweite Funktion anwendbar ist, in Abhängigkeit der Phasenverschiebung zwischen beginnender Einatmung und beginnendem Anstieg der den intrathorakalen Druck ITP zumindest näherungsweise wiedergebenden zweiten Meßgröße erfolgt, dergestalt,
daß für eine unter einem Schwellenwert liegende Phasenverschiebung zwischen beginnender Einatmung und beginnendem Anstieg der zweiten Meßgröße künstliche Beatmung anzunehmen ist,
und für eine über dem Schwellenwert liegende Phasenverschiebung zwischen beginnender Einatmung und beginnendem Anstieg der zweiten Meßgröße Spontanatmung anzunehmen ist.

26. Vorrichtung gemäß einem der Ansprüche 11-25, **dadurch gekennzeichnet, daß** die dritte Meßgröße zumindest näherungsweise der Thoraxexpansion des Patienten entspricht.

27. Vorrichtung gemäß Anspruch 26, **dadurch gekennzeichnet, daß** der dritte Eingangskanal (5c) zum Abfragen einer Dehnungsmeßbrücke (DMS) oder eines Impedanzmeßsensors ausgelegt ist.

28. Vorrichtung gemäß einem der Ansprüche 11 -25, **dadurch gekennzeichnet, daß** die dritte Meßgröße zumindest näherungsweise dem Beatmungsdruck oder dem Volumenstrom der Atemluft des Patienten entspricht.

29. Vorrichtung gemäß einem der Ansprüche 11 -28, **dadurch gekennzeichnet, daß** der zweite Eingangskanal (5b) zum Abfragen eines Druckmeßumformersignals ausgelegt ist,
und die zweite Meßgröße zumindest näherungsweise dem zentralvenösen Druck des Patienten entspricht.

Fig. 1 (Hintergrund der Erfindung)

Fig. 2

Fig. 3

*Fig. 4a*

*Fig. 4b*

*Fig. 5a*

*Fig. 5b*